Europäisches Patentamt

European Patent Office

Office européen des brevets

(11) Publication number: 0 310 586
A1

# EUROPEAN PATENT APPLICATION

(21) Application number: 88870152.1

(22) Date of filing: 23.09.88

(51) Int. Cl.⁴: $C\ 12\ N\ 15/00$

(30) Priority: 28.09.87 US 101463

(43) Date of publication of application:
05.04.89 Bulletin 89/14

(84) Designated Contracting States:
AT BE CH DE ES FR GB GR IT LI LU NL SE

(71) Applicant: Smith Kline - RIT Société anonyme dite:
rue du Tilleul, 13
B-1320 Genval (Rixensart) (BE)

(72) Inventor: Boeke, Jef Daniel
1 South Beechwood Avenue
Catonsville Maryland 21228 (US)

Dewerchin, Marianne G.
Rue de la Montagne, 19
B-1460 Ittre (BE)

Jacobs, Eric J.J.
Avenue des Erables, 5
B-1320 Genval (BE)

(74) Representative: Tasset, Gérard
SMITHKLINE - RIT rue de l'Institut, 89
B-1330 Rixensart (BE)

(54) Stable genomic integration and expression of a foreign coding sequence in yeast.

(57) A method of effecting reverse transcriptase mediated transposition of a foreign coding sequence into a host yeast chromosome by using a Ty1 or Ty2 based vector containing a foreign coding sequence controlled by a strong yeast promoter.

EP 0 310 586 A1

Description

## STABLE GENOMIC INTEGRATION AND EXPRESSION OF A FOREIGN CODING SEQUENCE IN YEAST

### BACKGROUND OF THE INVENTION

This invention relates to a method of effecting reverse transcriptase mediated transposition into host yeast chromosomes by using a Ty based vector containing a foreign coding sequence controlled by a strong yeast promoter.

Boeke et al., Cell, 40, 491-500 (1985) have developed an efficient assay for Ty1 transposition involving a functional Ty1 element. A GALI promoter controlled Ty1 element (TyH3) was included into a multicopy 2 micron, URA3 plasmid, and transposition is induced when the cells are grown on galactose as carbon source at low temperature. Furthermore, a short DNA sequence (i.e., a 40 bp lacO fragment) was inserted into a Ty1 non essential region (near the 3' LTR) without significantly inhibiting the transposition.

Boeke et al., Cell, 40, 491-500 (1985), describe a system for studying Ty1 transposition in which a genetically tagged Ty1 element (i.e., a Ty1 element containing a lacO segment) is fused to the controllable GAL1 promoter of yeast, and upon galactose induction, the frequency of transposition of both the marked element and genomic Ty1 elements dramatically increases. Boeke et al. also describe that an intron (the 398 nucleotide yeast ribosomal protein 51 gene intron, rp51) implanted in the donor Ty1 is correctly spliced from the Ty1 element during transposition, proving that Ty1 RNA is the intermediate in transposition. Furthermore, Boeke et al. conclude that the movement of sequence polymorphisms between the LTRs that occur during transposition follows the model of retroviral reverse transcription. Boeke et al make no observations or suggestions regarding transcription of the genetically tagged Ty1 element.

Garfinkel et al., Cell, 42, 507-517 (1985), describe that a Ty1 encoded reverse transcriptase activity is indeed present in transposition-induced cells which contain a plasmid that has a Ty1 element (TyH3) containing a lacO segment under the control of the GAL1 promoter.

Boeke et al., Twelfth International Conference On Yeast Genetics and Molecular Biology, Edinburgh, Scotland, September 17-21, 1984, describe gene fusions on 2 micron vectors (pGTy173 and pGTyH3) in which the GAL1 promoter elements promote the transcription of two different Ty elements, Ty173 (a Ty element isolated as a knock-out mutation in the LYS2 gene) and TyH3 (a Ty element isolated as a revertant of his 3Δ4 (a promoter deletion). Boeke et al. also describe that cells transformed with pGTyH3 and pGTy173 grow normally on glucose-containing medium, but when the GAL1 promoter is induced, growth is inhibited slightly by pGTy173 and very strongly with pGTyH3. Boeke et al. also describe an insertion mutation of the pGTyH3 plasmid in which the Km$^r$ gene from Tn903 was inserted at 2 points corresponding to the open reading frames of the Ty element. Boeke et al. make no observations or suggestions regarding (a) transposition of pGTyH3, pGTy173 or the pGTyH3 internal insertion mutant plasmids, or (b) transcription of the Km$^r$ gene by cells transformed with the pGTyH3 internal insertion mutant plasmid.

It has now been discovered that by using a vector system comprising a modified S. cerevisiae Ty1 or Ty2 element, reverse transcriptase mediated transposition and stable integration of a foreign coding sequence controlled by appropriate yeast promoters into the yeast chromosomes can be induced. Such modified Ty1 or Ty2 elements contain expression cassettes having a strong yeast promoter and a foreign gene of interest (e.g., pARG3-GALK or pCUP1-GALK) having sizes as high as 2 Kilobases (kb) can be transposed relatively efficiently. In fact, several transposition events per genome can be obtained in a single experiment (as observed by screening after induction of transposition). Higher copy numbers of integrated vectors can be obtained after multiple transposition cycles. The copies are stably integrated and disseminated (not tandemly repeated) into the chromosomes, allowing extensive growth of the host in the absence of selective pressure. On the other hand, it is important to note that such integrated vectors are homgeneously distributed into the cell population, while plasmids usually have a segregation bias. Thus, the method of this invention permits the synthesis of the foreign coding sequence product of interest at a well defined level and with the same efficiency in each cell of the population. The method of this invention could also permit the introduction of the expression cassette at a high copy number in the genome since most of the S. cerevisiae genomic sequences are not essential in classical growth conditions.

### SUMMARY OF THE INVENTION

This invention relates to a vector comprising a promoter, preferably an inducible promoter, operatively linked to a functional Ty1 or Ty2 element, wherein such Ty1 or Ty2 element comprises an expression cassette containing a strong yeast promoter operatively linked to a foreign coding sequence.

This invention also relates to a host yeast cell, preferably S. cerevisiae, transformed with such vector; and this invention also relates to a method of effecting stable genomic integration and expression of the foreign coding sequence which comprises culturing such transformed host yeast cell, preferably S. cerevisiae, under conditions which permit the strong yeast promoter to function.

By the term "expression cassette" is meant any discrete region of DNA which functions in yeast as a complete gene expression unit. By the term "complete gene expression unit" is meant a structural gene and the promoter and regulatory regions required for its transcription and translation.

By the term "promoter" is meant any region upstream of a structural gene's coding sequence which permits binding of RNA polymerase and transcription of the coding sequence to occur. By "regulatory region" is meant any region which regulates transcription of the gene. By "structural gene" is meant a coding sequence which serves to be the template for the synthesis of RNA and allows the synthesis of the protein of interest in yeast.

By the term "inducible promoter" is meant a promoter which is functional in a host yeast cell, such as S. cerevisiae, and whose activity is controlled by an external signal or condition, e.g., temperature change, presence or absence of a nutrient, etc.

By the term "functional Ty1 or Ty2 element" is meant any derivative of a naturally occurring Ty1 or Ty2 element which comprises a strong yeast promoter operatively linked to a coding sequence of a foreign gene and still retaining its ability to be transposed into the genome of a host yeast cell, such as S. cerevisiae, transformed with such element.

By the term "operatively linked" is meant that a promoter and a coding sequence are fused in such a way to permit transcription and translation of the coding sequence.

By the term "strong yeast promoter" is meant a promoter which is functional in a host yeast cell, such as S. cerevisiae, and which will allow the production of high expression levels of heterologous proteins in yeast.

By the term "foreign coding sequence" is meant a coding sequence which does not naturally occur within the host yeast cell genome.

By the term "transposition" is meant the reverse transcriptase mediated nonhomologous insertion of the functional Ty1 or Ty2 element of the vector of this invention into the host yeast cell chromosomes at a location unoccupied by a previously existing Ty element. The mechanism by which the transposition actually occurs is described in greater detail by Boeke, et al., Cell, 40, 491-500 (1985).

By the term "transcription run through" is meant complete transcription of the functional Ty1 or Ty2 element of the vector of this invention.


## BRIEF DESCRIPTION OF THE DRAWINGS


Figure 1(a) shows the adapted E. coli GALK structural gene used for constructing the GALK expression cassettes used in the vectors described in the Examples. Such GALK contains a modified amino terminal region but encodes an active galactokinase.

Figure 1(b) shows the 5' regions of the CAR1 (Arginase), ARG3 (Ornithine transcarbamylase) and CUP1 (Metallothionein) genes. Each 5' region contains a BamHI site after the ATG thereby allowing functional association with GALK.

Figure 1(c) shows the association of the CAR1, ARG3 and CUP1 promoter regions with the GALK coding sequence using the BamHI sites to create the expression cassettes.

Figure 2(a), Part A, shows the organization of pJef1267 which contains a pGAL1-TY1 construction.

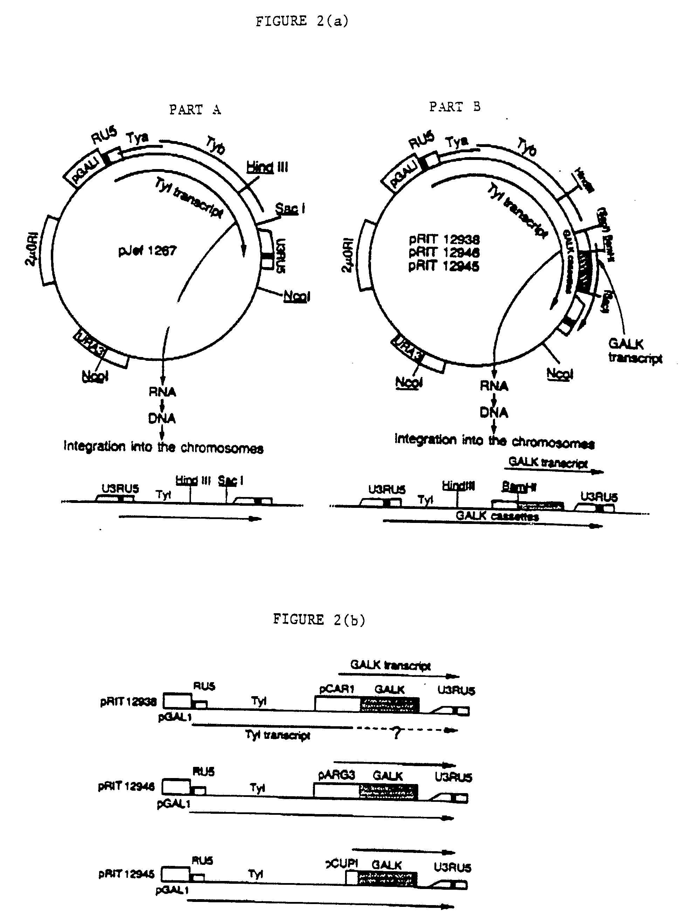

Figure 2(a), Part B, shows the organization of pRIT12938, pRIT12946 and pRIT12945 which are derivatives of pJef1267 and which contain expression cassettes comprising CAR1 promoter-GALK (pRIT12938), ARG3 promoter-GALK (pRIT12946) and CUP1 promoter-GALK (pRIT12945) introduced into the SacI restriction site of pJef1267.

Figure 2(b) shows the structure of the pGAL1-Ty1 constructions containing the GALK expression cassettes described in the Examples. The arrows represent the orientations of the Ty and GALK transcripts.


## DETAILED DESCRIPTION OF THE INVENTION


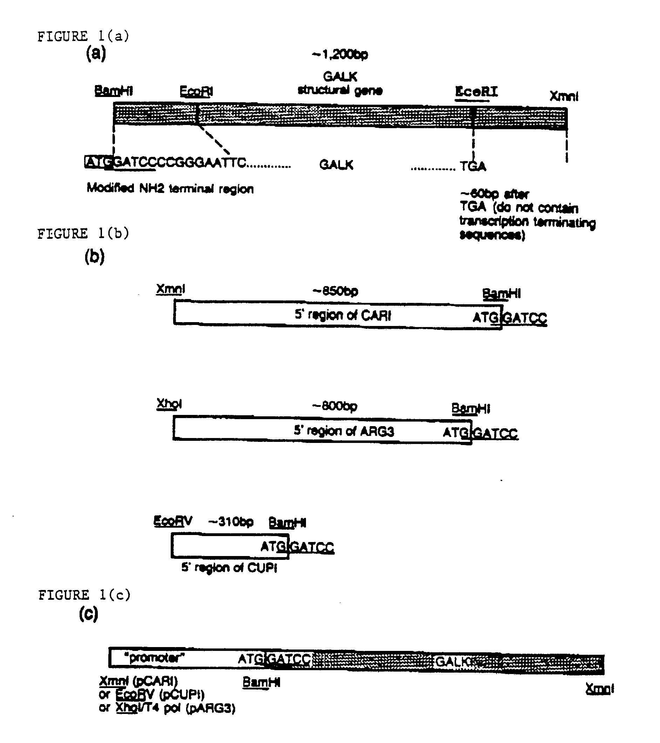

Ty elements are a family of naturally occurring transposable elements from the yeast Saccharomyces cerevisiae that are structurally similar to retroviral proviruses and the copia element family of Drosophila. For a detailed review of such Ty elements, see, e.g., Mellor et al., Yeast, 2, 145-152 (1986). Such Ty elements may be isolated from a strain of S. cerevisiae by known techniques. Numerous strains of S. cerevisiae are available from public depositories, e.g., the American Type Culture Collection, Rockville, Maryland, U.S.A. The number and distribution of these elements varies from strain to strain. These elements are approximately 5.9 kilobases (kb) in length. Terminally repeated delta sequences or direct long terminal repeats (LTRS) of approximately 335 base pairs (bp) surround an internal region of 5.3 kb. Ty elements are transcribed from LTR to LTR forming an RNA that is terminally redundant for 45 nucleotides. This structure is identical to the overall structure of retroviral genomic RNA. Ty elements contain a sequence that could serve as a tRNA primer binding site adjacent to the 5' LTR and an oligo-purine tract which could prime second-strand synthesis adjacent to the 3' LTR. Moreover, the functional organization of Ty elements is similar to the organization of the gag-pol region of retroviruses. In Ty elements, there are two overlapping open reading frames; i.e., tya, which is probably equivalent to the retroviral gag gene and specifies a protein with homology to DNA binding proteins; and tyb, which encodes a protein with homology to the protease, integrase, and reverse transcriptase regions of the retroviral pol gene. The gene product of tyb is thought to be synthesized as a tya-tyb fusion protein resulting from a specific frameshift at the end of tya that puts tyb in frame with tya. There are two classes of Ty elements, i.e., Ty1 and Ty2 which differ essentially by two large internal substitutions. The construction of the efficient Ty1 or Ty2 based expression vectors of this invention requires the use of strong yeast promoters which do not contain transcription terminator-like elements or any other sequences which would prohibit transcription run through so that synthesis of the large RNA which is necessary for the transposition of these constructions is not interfered with (Figure 1a). Indeed, in such a system, the large RNA which is produced when the inducible promoter is induced is used as a mRNA for the synthesis of the transposition machinery and is also the template in the transposition process.

This invention relates to a vector comprising a promoter operatively linked to a functional Ty1 or Ty2 element, wherein such Ty1 or Ty2 element comprises an expression cassette containing a strong yeast promoter operatively linked to a foreign coding sequence. Such a vector can be constructed by conventional methods well known in the art. Preferably, a Ty1 element is employed to construct such vector and such Ty1 element employed is derived from TyH3 which is described in detail in Boeke et. al., Cell, 40, 491-500 (1985). As stated above, the vector of this invention comprises a promoter operatively linked to a functional Ty1 or Ty2 element. Any inducible or non-inducible promoter which is functional in the host yeast cell in which the vector will transformed may be employed. Preferably, the promoter is inducible. Also, preferably, the promoter employed is the GAL1 promoter described in U.S. Patent 4,661,454 issued April 28, 1987. As stated above, the functional Ty1 or Ty2 element of the vector of this invention comprises an expression cassette containing a strong yeast promoter operatively linked to a foriegn coding sequence. Any strong yeast promoter, whether inducible or non-inducible, which will be functional in the host yeast cell in which the vector will be transformed may be employed provided that such promoter per mits transcription run through of the foreign coding sequence. Preferably, the strong yeast promoter is from the S. cerevisiae ARG3 gene or the S. cerevisiae CUP1 gene. Any foreign coding sequence which can be transcribed and translated in the host yeast cell to produce a protein with biological activity may be employed. Preferably, such protein has therapeutic biological activity.

This invention also relates to a host yeast cell transformed with the vector of this invention. Such transformation can be accomplished by conventional techniques, such as by the method of Ito et. al., J. Bacteriol., 153, 163-168 (1983). Preferably, the host yeast cell belongs to the genus Saccharomyces, most preferably it belongs to the species S. cerevisiae.

This invention also relates to a method of effecting stable genomic integration and expression of a foreign coding sequence which comprises culturing the transformed host of this invention under conditions which will permit the strong yeast promoter comprised by the vector of the invention to function. For example, if the strong yeast promoter employed is an inducible promoter, such as GAL1, then the host yeast cell should be cultured in a medium containing sufficient galactose to permit the induction of the GAL1 promoter. Similarly, if the strong yeast promoter employed is the S. cerevisiae ARG3 promoter, the culture medium employed should contain sufficient NH4$^+$ to permit the induction of the ARG3 promoter.

All strong yeast promoter sequences are not free from such terminator-like elements or other sequences which would prohibit transcription run through such as sequences which interact with regulatory proteins. For example, the URA3 promoter region contains a strong bidirectional terminator (between bases 45 and 155 upstream to the first ATG). See, Yarger et al., Mol. Cell. Biol., 6, 1095 (1986). In the case of the CARI promoter, the CARI 5′ region described in the Examples, one or some of these possible interferences may be occurring.

In the following Examples, specific embodiments of the invention are more fully disclosed. These Examples are intended to be illustrative of the subject invention and should not be construed as limiting its scope.

## EXAMPLES

### MATERIALS AND METHODS

#### 15. a. Strains and plasmids

- Two strains of S. cerevisiae were used in the Examples; GRF167 (his3 Δ200, ura3-167. and JWG2-1D (trpl ura 3 gall Δ)

- The plasmid pJef724 is identical to pGTyH3, described previously. See, Boeke et al., Cell, 40, 491-500 (1985). pJef1267 derives from pJef724 (introduction of a SacI site in place of the Ty1 3′ BglII site). pJef724 is a 2 micron based plasmid. All Ty-based plasmids used in transposition experiments described in these Examples are derivatives of pJef1267, in which foreign DNA coding sequences (GALK cassettes) are introduced in the SacI restriction site, as described in detail, infra. The GALK expression cassettes were also introduced into a low copy number plasmid YCp50. See, Johnston and Davis, Mol. Cell. Biol., 4, 1440 (1984). HindIII-NcoI (T4 polymerase treated) fragments from pRIT12938, pRIT12945, PRIT12946 were transferred into the YCp50 plasmid digested by HindIII-BamHI (T4 polymerase treated). See, Figure 2a. XY12 is a derivative of PFL1 (Chevallier et al., Gene, 11, 11-19 (1980)) and contains the pARG3-GALK expression cassette with the transcription termination sequence of the ARG3 gene.

4

**b. Culture media**

YEPD-rich media (2 % each yeast extract, peptone, glucose or galactose) and YNB minimal media (0.67 % yeast nitrogen base without amino acids, with or without ammonium sulfate) were used for growth of S. cerevisiae strains. YNB without ammonium sulfate was supplemented with arginine 1 mg/ml as sole nitrogen source. YNB media were supplemented with glucose 2 % or galactose 2 % as sole carbon sources.

Fluoroorotic-acid containing medium is described in Boeke et al., (Mol. Gen. Genet., 197, 345-346 (1984).

**c. Transformation**

E. coli transformation with plasmid DNA was carried out according to Cohen et al., Proc. Natl. Acad. Sci., U.S.A., 69, 2110-2114 (1972). Transformation of yeast was as described by Ito et al., J. Bacteriol., 153, 163-168 (1983).

**d. Analysis of S. cerevisiae genomic DNA sequences**

(1) Total genomic DNA of S. cerevisiae was isolated according to Davis et al., Methods in Enzymology, 65, 404-411 (1980), with minor modifications. DNA restrictions, electrophoresis and Southern blot analysis were performed as described in Maniatis et al., Molecular Cloning, A Laboratory Manual, Cold Spring Harbor Laboratory (1982).

(2) A 1.1 kb GALK DNA fragment is used for detection of transposed expression cassettes. See, Figure 1(a), 1(b) and 1(c). A 420 bp specific Ty1 DNA fragment is used for analysis of resident Ty1 transposition (BglII-EcoRI fragment). The 850 bp XmnI-BamHI CARI DNA fragment is used as a control for total digestions of genomic DNA in Southern blots (DNA analysis of the strains having pARG3-GALK or pCUP1-GALK cassettes integrated in their genome).

**e. Determination of galactokinase specific activities**

The galactokinase (GALK) enzymatic activities were measured as described in Rymond et al., Gene, 25, 249-262 (1983), and normalized to the total protein levels, quantitated by the Folin method of Lowry et al., J. Biol. Chem., 193, 265-275 (1951).

**f. Transposition experiments (See also Figures 2a and 2b.)**

Using the plasmid pJef724 (or derivatives described, infra) transposition was performed as follows:

(1) transformation of the S. cerevisiae recipient strain (complementation of the ura3 mutation) on YNB glucose 2 % medium at 30° C.

(2) Purification of the transformants (isolated colonies).

(3) Induction of transposition. The cells are growing 5 days at 22° C on YNB galactose 2 % medium.

(4) Selection of ura3 colonies (strains having lost the plasmid, on fluoroorotic acid containing medium).

(5) Purification of ura3 strains (isolated colonies on YEPD medium)

(6) Search for ura3 strains growing on galactose as carbon source, if the recipient strain is gall (JWG2-1D). Analysis of the total genomic DNA by hybridization with a GALK specific DNA probe.

**3. Construction of the vectors of this invention and Results of Transposition experiments**

A vector of this invention may be prepared as a derivative of the pGTyH3 (pJef724) construction described in Boeke et al., Cell, 40, 491-500 (1985), which is hereby incorporated by reference. For example, in pGTyH3, a strong yeast promoter, such as the GAL1 promoter, replaces the U3 region of the Ty1 5′ LTR in such a way that the synthesis of functional Ty1 transcripts is controlled by galactose induction. In each vector of this invention, the foreign coding sequence is inserted into a non-essential Ty1 region, so that the Ty1 element remains functional. For example, a synthetic DNA sequence containing the SacI restriction site was first introduced by conventional methods into the 3′ Bg III site of TyH3) to create pJef1267. The foreign coding sequence was inserted into the unique SacI restriction site by conventional methods. See, Figure 2. The E. coli galactokinase structural gene (GALK) was selected as an example of a foreign coding sequence (and can also be used as a selective marker, thus allowing the selection for transposition events in a S. cerevisiae gall recipient strain).

The GALK coding sequence used contains a modified NH$_2$ terminal region, but encodes an active galactokinase. See, Rymond et al., Gene, 25, 249-262 (1983). Three different S. cerevisiae promoter regions were fused to GALK in the Ty1 vectors, i.e., CAR1, ARG3 and CUP1. The sequences of these promoter regions correspond to the regions 5′ upstream from the ATG of the corresponding S. cerevisiae genes and were adapted with BamHI restriction sites allowing fucntional associations with GALK. See, Figure 2.

The 5′ region of the CAR1 gene (arginase structural gene) contains at least two regulatory regions corresponding to two different controls, i.e. nitrogen catabolite repression (Cunin et al., Mol. Cell. Biol., 5, 1339-1348 (1985) and arginine specific induction (Messenguy and Dubois, Mol. Gen. Genet., 189, 148-156 (1983). The 5′ region of the CAR1 gene is isolated as a 820 bp XmnI-BamHI DNA fragment which contains the promoter region and most of the regulatory sequences. The control region involved in nitrogen catabolite repression is not completely included.

The ARG3 (ornithine transcarbamylase structural gene) 5′ sequence contains two regulatory regions. ARG3 is regulated by the "general amino acid control" and the Arginine specific control described by Messenguy et al., (1983), cited above, and Crabeel et al., Mol. Cell Biol., 2, 1339-1348 (1985). The 5' region of ARG3 is contained in a 800 bp XhoI-BamHI DNA fragment which contains the promoter and all regulatory sequences. See Figure 1(b).

The CUP1 (S. cerevisiae metallothionine structural gene) 5' sequence contains the DNA regions involved in copper induction. The 300 bp EcoRV-BamHI fragment of the CUP1 gene used contains these DNA regions.

These three promoters, i.e., CARI, ARG3 and CUP1 were associated with GALK by using the BamHI site with the GALK gene, and the resulting DNA fragments were inserted into the SacI unique site of pJef1267 (after treatment with T4 polymerase). The resulting multicopy plasmids i.e., pRIT12938, Figure 2(b)); RIT12946 (Figure 2(b)) and pRIT12945 (Figure 2(b)) were used in transposition experiments in a GAL⁺ ura3 S. cerevisiae strain, and also in a gall ura3 S. cerevisiae strain (JWG2-1D) because the E. coli galactokinase can complement the gall mutation (growth on galactose (2%) as a carbon source).

Comparisons of growth rates on galactose of such S. cerevisiae strains transformed with the vectors described herein involving low copy number YCp50 constructions containing the same expression cassettes were conducted in the different regulatory conditions.

The complementation efficiencies (growth rates on galactose) are related to the regulatory conditions, and copy numbers of the GALK expression cassettes. As can be seen in Table 1, the CAR1 promoter is more efficient on arginine as the nitrogen source (specific induction, no nitrogen catabolite repression), the ARG3 promoter is more efficient when $NH_4^+$ is used as nitrogen source (no repression in the absence of arginine) and the CUP1 promoter is more efficient in the presence of copper (specific induction). These effects are more pronounced when low copy number plasmids are used.

Most of the transposition experiments were conducted in the gall $\Delta$ ura3 trpl strain, JWG2-1D, because after induction of transposition and plasmid curing, strains having supported one or several transposition events (i.e., having one or several integrated copies of the expression cassette into the chromosomes) can be easily identified by their capacity to grow on galactose as carbon source. It is interesting to note that the growth rates of such strains are similar to the growth rates of the JWG2-1D strain containing the corresponding GALK cassettes located on a low copy number YCp50 plasmid.

The results of a series of transposition experiments are summarized in Table 2 which shows the transposition efficiencies. Other experiments were conducted at a lower temperature (16° C), with similar results (no significantly increased efficiencies).

Using the three expression cassettes (pCAR1-GALK, pARG3-GALK and pCUP1-GALK), transposition assays were conducted in the different regulatory conditions ($NH_4^+$ or arginine as nitrogen sources in the case of CAR1 and ARG3 promoters, and in the presence or absence of copper in the case of the CUP1 promoter). The plasmid pJef724 which is equivalent to pJef1267 (original BglII site in place of the SacI site) was used as a control (i.e., no foreign DNA was inserted into Ty1) in transposition experiments into the GAL⁺ strain (GRF167-his3 ura3). The plasmid pRIT12938 containing the Ty1 (pCAR1-GALK) construction was introduced into the same GAL⁺ strain and into the gall $\Delta$ strain, JWG2-1D (trpl ura3 gall$\Delta$).

In the case of the CAR1-GALK cassette, pRIT12938, more than 100 strains were analyzed after induction of transposition and plasmid curing, but GALK transposition was never observed after analysis of the capacity of the JWG2-1D gall $\Delta$ strain, to grow on galactose with arginine as its nitrogen source, and DNA analysis using a GALK specific DNA probe. See, Table 2. However, the GAL1-Tyl-CAR1-GALK construction of pRIT12938 is efficient regarding the synthesis of the transposition machinery, because an induction of resident Ty1 transposition using the plasmid pRIT12938 was observed, i.e., at least 40% of strains having supported one or many transposition events. See, Table 2.

These results indicate that the CAR1-GALK hybrid gene is not compatible with efficient reverse transcriptase mediated transposition, probably because the CAR1 promoter does not allow the transcription runthrough (or allows it only with a low frequency) which is necessary for the transposition of the vectors of this invention, possibly because this promoter contains a transcriptional terminator-like sequence. The size of the embedded expression cassette is not incompatible with transposition, because foreign DNA sequences having similar lengths can be efficiently transposed as described, infra, in more detail. The results of evaluation of Ty1 vectors containing the ARG3-GALK and CUP1-GALK hybrid genes revealed that in such vectors the transposition is relatively efficient. In the experiments reported in the Table 2, 15 to 44% of the strains analysed after induction of transposition and plasmid curing have supported at least one transposition event (integration of the modified Ty1 into the chromosomes). However, transposition frequencies of such modified Ty1 element are approximately 10 times lower than Ty1 transposition frequencies obtained using the original pGAL1 Ty1 construction (i.e., transposition of the Ty1 element controlled by pGAL1 + chromosomal Ty1 elements).

After induction of transposition and plasmid curing, these strains were analysed for their capacity to grow on galactose as carbon source, with $NH_4^+$ as nitrogen source for optimal expression of the ARG3 promoter and in the presence of copper in the case of pCUP1-GALK for optimal expression of the CUP1 promoter. In these conditions, the capacity to grow on galactose was always correlated with the presence of GALK copies integrated into the chromosomes as determined by DNA analysis using a GALK specific probe. Growth rates on galactose of strains having one or some integrated vector copies and of the strain containing the corresponding low copy number construction on YCp50 are similar. In such experiments, strains supporting one, two or three transposition events (1, 2 or 3 copies integrated) were easily obtained with copy numbers being estimated by the number of BamHI genomic fragments recognized with the GALK probe (there is only one BamHI site in the Ty1 constructions.)

In these experiments, significant variations in transposition efficiencies with the ARG3-GALK or CUP1-GALK cassettes in the different regulatory conditions were not observed, particularly in the case of the pCUP1-GALK plasmid pRIT 12945, where a strong variation in growth rate on galactose resulting from variation in galactokinase synthesis (due to absence of copper versus presence of copper) did not significantly influence the transposition efficiency in the gallΔ strain JWG2-1D (See, Table 2).

Regarding the expression of the GALK cassettes inserted into Ty1, as shown in Table 3, it appears that the influence of induction of the GAL1 promoter on GALK expression is weak or nonexistent. Indeed, strains containing pRIT12946 (ARG3-GALK cassette) synthesize similar amounts of galactokinase when they are growing on glucose (no expression of Ty1) or galactose (induction of Ty1 expression) as carbon sources, and the galactokinase specific activities are of the same order as with usual 2 micron plasmids wherein ARG3-GALK is not inserted into a Ty1 element). On the other hand, strains having one pARG3-GALK copy integrated into the chromosome synthesize similar galactokinase amounts as the same strain containing a low copy number plasmid with the same expression cassette.

To increase the efficiency of the method of this invention, appropriate selection markers, such as the S. cerevisiae CUP1 (metallothienein) gene, could be introduced into the vectors of this invention in addition to the expression cassettes, thereby allowing the selection of strains supporting high numbers of transposition events in a simple experiment. If the CUP1 gene is employed as the selection marker, the recipient strain used must be a Cup1ˢ or Cup1Δ strain. To avoid a possible decrease in transposition efficiency if employing a selection marker, it may be useful to reduce the Ty sequences of the vector to the minimal size allowing efficient encapsidation of the transcript, the transposition machinery being produced using a "helper" plasmid. For example, the 5' sequences extending to the first BglII site of Ty1 are probably sufficient. Such a vector will be identical to, for example, pRIT12946, except that the internal Ty1 sequence extending from the 5' BglII site to the point corresponding to the SacI site on the Ty1 element will be absent. Such a vector probably should be used in connection with a "helper" Ty element which has its 3' LTR replaced by another transcription terminating sequence but which is controlled by an inducible promoter (e.g., GAL1). The "helper" element could be part of a plasmid independent of the plasmid of the invention. The "helper" Ty element does not transpose but produces the Tya and Tyb proteins required by the plasmid of this invention for transposition. Also, the expression level of the Ty vectors could be increased by modifying or changing the GAL1 promoter, e.g., replacement with CUP1 5' sequences.

The vector system of this invention could offer many advantages as compared to episomes. The vector copies are stably integrated and disseminated (not tandemly repeated) into the chromosomes, allowing growth in the absence of selective pressure. Also, integrated cassettes are homogeneously distributed into the cell population, while plasmids usually have a segregation bias. Thus, the method of this invention allows the synthesis of the gene product at a well defined level and with the same efficiency in each cell of the population.

The following Table 1 shows the growth properties of the gall strain, JWG2-1D, containing the multicopy number plasmids pRIT12946, pRIT12938 or pRIT12945, or the low copy number plasmids derived from YCp50, i.e., pRIT 12947, pRIT12948 or pRIT12949.

The following Table 2 shows the transposition frequencies of GALK expression cassettes under different growth conditions.

The following Table 3 shows the levels of galactokinase produced by JWG2-1D strains carrying the pARG3-GALK expression cassette on a plasmid derived from pJef1267 or YCp50 or having one (TY1-pARG3-GALK) transposon integrated into the S. cerevisiae host chromosome.

Growth properties (replica plating on galactose 2 X media) of the gal 1 strain JWG2-1D containing the multicopy plasmids pRIT12938, pRIT12946, pRIT12945 and the low copy number plasmids pRIT12947, pRIT12948, pRIT12949 (see fig. 2). CAR1 promoter is induced on Arginine medium and not on $NH4^+$ medium. ARG3 promoter is more efficient on $NH4^+$ medium than on arginine medium. CUP1 promoter is more efficient in the presence of $Cu2^+$ in the medium. These effects are more pronounced with low copy number plasmids.

| PLASMID | CASSETTES | GROWTH RATES | | | |
|---|---|---|---|---|---|
| | | Gal 2X $NH4^+$ | Gal 2X ARG 1mg/ml | Gal 2 X $NH4^+$ | |
| | | | | + CuSO4 0,06mM | + CuSO4 0,6 mM |
| pRIT12938 (2μ) | pCAR1-GALK | ± | + | | |
| pRIT12947 (YCp50) | " | − | ± | | |
| pRIT12946 (2μ) | pARG3-GALK | ++ | + | | |
| pRIT12948 (YCp50) | " | + | ± | | |
| pRIT12945 (2μ) | pCUP1-GALK | ± | | ++ | ++ |
| pRIT12949 (YCp50) | " | − | | ± | + |
| pJKF1267 (2μ) | ---- | − | − | − | − |

TABLE 1

EP 0 310 586 A1

TABLE 2

Transposition frequencies of Ty1 and GALK expression cassettes embedded into Ty1) in different galactose 2 % growth media, at 22°C (5 days)

a) Ty1 transposition frequencies (in the case of pJef724, transposition of the Ty1 element controlled by pGAL1 + transposition of chromosomal Ty1 elements). The number of Ty1 transposition events is estimated by determination of the new genomic DNA fragments homologous to a Ty1 specific probe.

A : % of strains having supported at least one Ty1 transposition event.

B : mean number of Ty1 transposition events per genome.

| Strain | Plasmid | Ty1 transposition GAL2% + ARG1mg/ml | |
|---|---|---|---|
| | | A | B |
| GRF167 (his3 ura3 Gal+) | pJef724 | 60%-80% | 1-2 |
| GRF167 (his3 ura3 Gal+) | pRIT12938 GALK transposition was never observed | 40%-50% | 0,6 |

b) Transposition frequencies of the GALK expression cassettes (embedded into the Ty1 element), in the different growth conditions.

A : % of strains having supported at least one transposition event of the GALK expression cassette.

B : mean numbers of transposition events of the expression cassette per genome. The copy numbers are estimated by determination of the number of BamHI genomic DNA fragments homologous to GALK, by Southern blot

| Strain | Plasmid | GAL2% NH4+ | | GAL2% ARG | | GAL2% NH4+ +CuSO4 | |
|---|---|---|---|---|---|---|---|
| | | A | B | A | B | A | B |
| JWG2-1D (trp1,ura3,gal1 ) | pRIT12938 | 0% GalK transposition was never observed | 0 | 0% | 0 | | |
| JWG2-1D (trp1,ura3,gal1 ) | pRIT12946 | 17% | 0,19 | 44% | 0.48 | | |
| JWG2-1D (trp1,ura3,gal1 ) | pRIT12945 | 15%-18% | 0,17-0,20 | 18% | | 15%-21% | 0,17-0,27 |

EP 0 310 586 A1

TABLE 3

Galactokinase levels of JWC2-1 D strains carrying the pARG3-GALK expression cassette on a multicopy (2µ) plasmid, on a low copy number plasmid (YCp50) or having one [Ty1-pARG3-GALK] transposon integrated into the chromosomes. The 2µ plasmid pRIT12946 contains the pARG3-GALK cassette into the pGAL1-Ty1 construction. GALK expression was compared on glucose or galactose as carbon sources (respectively no Ty1 expression or Ty1 expression induced). In YCp50 vectors, the GAL1 promoter is absent.

| Plasmid | Carbon and Nitrogen sources | GALK specific activity (nM galactose/h ng prot) |
|---|---|---|
| XY12 (conventional 2µ plasmid containing ARG3-GALK) | NH4$^+$ glucose 2% | 93 000 |
| pRIT12946 (2µ) | NH4$^+$ glucose 2% | 64 500 |
| pRIT12946 (2µ) | NH4$^+$ galactose 2% | 98 000 · |
| pRIT12948 (YCp50) | NH4$^+$ glucose 2% | 22 200 |
| pRIT12948 (YCp50) | NH4$^+$ galactose 2% | 29 600 |
| pJef1267 | NH4$^+$ glucose 2% | Not detected |
| 330 (transposed cassette) | NH4$^+$ glucose 2% | 12 400 |
| 335 (transposed cassette) | NH4$^+$ glucose 2% | 12 300 |
| 342 (transposed cassette) | NH4$^+$ glucose 2% | 23 800 |
| 340 (transposed cassette) | NH4 glucose 2 % | 20 400 |

10

While the above descriptions and examples fully describe the invention and the preferred embodiments thereof, it is understood that the invention is not limited to the particular disclosed embodiments. Thus, the invention includes all embodiments coming within the scope of the following claims.

**Claims**

1. A vector comprising a promoter operatively linked to a functional Ty1 or Ty2 element wherein such Ty1 or Ty2 element comprises an expression cassette containing a strong yeast promoter operatively linked to a foreign coding sequence.

2. The vector of Claim 1 wherein the promoter is GAL1.

3. The vector of Claim 1 wherein the vector comprises a functional Ty1 element.

4. The vector of Claim 3 wherein the functional Ty1 element is a derivative of TyH3.

5. The vector of Claim 1 wherein the vector additionally contains a selection marker.

6. The vector of Claim 5 wherein the selection marker is the S. cerevisiae CUP1 gene.

7. The vector of Claim 1 wherein the foreign coding sequence codes for a protein with biological activity.

8. The vector of Claim 7 wherein the protein has therapeutic biological activity.

9. The vector of Claim 1 wherein the strong yeast promoter is from the S. cerevisiae ARG3 gene.

10. The vector of Claim 1 wherein the strong yeast promoter is from the S. cerevisiae CUP1 gene.

11. The vector of Claim 1 which is a derivative of pJef1267 and comprises the expression cassette inserted in its SacI site.

12. A host yeast cell transformed with a vector wherein said vector comprises a promoter operatively linked to a functional Ty1 or Ty2 element wherein such Ty1 or Ty2 element comprises an expression cassette containing a strong yeast promoter operatively linked to a foreign coding sequence.

13. The host of Claim 12 which is belongs to the genus Saccharomyces.

14. The host of Claim 13 which belongs to the species S. cerevisiae.

15. The host of Claim 12 wherein the promoter is GAL1.

16. The host of Claim 12 wherein the vector comprises a functional Ty1 element.

17. The host of Claim 16 wherein the functional Ty1 element is a derivative of TyH3.

18. The host of Claim 12 wherein the expression cassette additionally contains a selection marker.

19. The host of Claim 18 wherein the selection marker is the S. cerevisiae CUP1 gene.

20. The host of Claim 12 wherein the foreign coding sequence codes for a protein with biological activity.

21. The host of Claim 20 wherein the protein has therapeutic biological activity.

22. The host of Claim 12 wherein the strong yeast promoter is from the S. cerevisiae ARG3 gene.

23. The host of Claim 12 wherein the strong yeast promoter is from the S. cerevisiae CUP1 gene.

24. The host of Claim 12 wherein the vector is a derivative of pJef1267 and comprises the expression cassette inserted in its SacI site.

25. A method of effecting stable genomic integration and expression of a foreign coding sequence which comprises culturing the host of Claim 12 under conditions which permit the strong yeast promoter to function.

26. The method of Claim 25 wherein the strong yeast promoter is an inducible promoter.

27. The method of Claim 26 wherein the inducible promoter is GAL1.

28. The method of Claim 26 wherein the inducible promoter is from the S. cerevisiae CUP1 gene.

29. The method of Claim 26 wherein the inducible promoter is from the S. cerevisiae ARG3 gene.

FIGURE 1(a)

**(a)**

~1,200bp

GALK structural gene

BamHI  EcoRI  EceRI  Xmnl

ATGGATCCCCGGGAATTC............  GALK  ............ TGA

Modified NH2 terminal region

~60bp after TGA (do not contain transcription terminating sequences)

FIGURE 1(b)

**(b)**

Xmnl  ~850bp  BamHI

5' region of CARI  ATGGATCC

Xhol  ~800bp  BamHI

5' region of ARG3  ATGGATCC

EcoRV  ~310bp  BamHI

ATGGATCC

5' region of CUPI

FIGURE 1(c)

**(c)**

"promoter"  ATGGATCC  GALK

Xmnl (pCARI)  BamHI  Xmnl
or EcoRV (pCUPI)
or Xhol/T4 pol (pARG3)

FIGURE 2(a)

PART A

PART B

FIGURE 2(b)

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,Y | CELL<br>volume 40, March 1985, Mass., USA, pages 491-500; J.D. BOEKE et al.: "Ty elements transpose through an RNA intermediate" * whole document * | 1-4,7,8,12-14, 16,17, 20,21, 25 | C 12 N 15/00 |
| Y | NUCLEIC ACIDS RESEARCH<br>volume 15, no. 18, 25th September 1987, Eynsham, Oxford, GB, pages 7571-7580; M.H. MALIM et al.: "The production of hybrid Ty:IFN virus-like particles in yeast" * whole document * | 1-4,7,8,12-14, 16,17, 20,21, 25 | |
| A | CHEMICAL ABSTRACTS<br>volume 104, no. 25, 23rd June 1986, Columbus, Ohio, US, page 161, column 1, abstract no. 220008q; G. TSCHUMPER et al.: "High frequency excision of Ty elements during transformation of yeast"; & NUCLEIC ACIDS-RES. 1986, volume 14, no. 7, pages 2988-3001 | 1,3,12, 16,25 | |
| A | CHEMICAL ABSTRACTS<br>volume 96, no. 15, 12th April 1982, Columbus, Ohio, US, page 147, column 2, abstract no. 116614q; S. SCHERER et al.: "Studies on the transposable element Ty1 of yeast. II. Recombination and expression of Ty1 and adjacent sequences"; & COLD SPRING HARBOR SYMP. QUANT. BIOL. 1981, volume 45, pages 584-591<br>---        -/- | 1,3,7, 12,16, 20,25 | TECHNICAL FIELDS SEARCHED (Int. Cl.4)<br><br>C 12 N 15/00 |

The present search report has been drawn up for all claims

| Place of search | Date of completion of the search | Examiner |
|---|---|---|
| BERLIN | 06-12-1988 | JULIA P. |

European Patent
Office

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS volume 103, no. 15, 14th October 1985, Columbus, Ohio, USA, page 195, column 2, abstract no. 117398x; D. BREILMANN et al.: "Gene conversion and reciprocal exchange in a Ty-mediated translocation in yeast"; & CURR. GENET. 1985, volume 9, no. 7, pages 553-560 | 1,12,25 | |
| A | EP-A-0 178 220  (INSTITUT PASTEUR) * whole document * | 1,12,25 | |
| A | US-A-4 670 388  (G.M. RUBIN et al.) * abstract; column 4, lines 49-56; column 6, lines 8-21,35-55; column 8, line 65 - column 9, line 2; claims * | 1 | |
| P,A | WO-A-8 801 646  (ALLELIX INC.) * abstract; page 2, lines 9-14; page 7, lines 16-25; page 14, line 7 - page 15, line 26; page 19, lines 12-28; page 62; examples 5,6; claims * | 1,12,25 | TECHNICAL FIELDS SEARCHED (Int. Cl.4) |
| A | EP-A-0 220 009  (E.I. DU PONT DE NEMOURS & CO.) * whole document * | 1,7,12, 25 | |

The present search report has been drawn up for all claims

| Place of search | Date of completion of the search | Examiner |
|---|---|---|
| BERLIN | 06-12-1988 | JULIA P. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P0401)